# EUROPÄISCHE PATENTSCHRIFT

(11) **EP 0 468 347 B1**
(45) Veröffentlichungstag und Bekanntmachung des Hinweises auf die Patenterteilung: **03.03.1993**
(21) Anmeldenummer: 91111904.8
(22) Anmeldetag: 17.07.1991
(51) Int. Cl.: A43B 3/24, A61F 5/01, A43B 3/26, A61F 13/04

(54) **Verbandsschuh**
Shoe for bandaged foot
Chaussure pour pied bandé

(30) Priorität: 21.07.1990 DE 9010863 U
(43) Veröffentlichungstag der Anmeldung: 29.01.1992
(73) Patentinhaber: Kühnreich, Heinz-Peter, D-53840 Troisdorf (DE)
(72) Erfinder: Kühnreich, Heinz-Peter, D-53840 Troisdorf (DE)
(74) Vertreter: Peerbooms, Rudolf, Dipl.-Phys.

(56) Entgegenhaltungen:
- GB-A- 2 168 234
- US-A- 2 236 367
- US-A- 2 438 711
- US-A- 3 417 408
- US-A- 4 300 294
- US-A- 4 314 412
- US-A- 4 575 954
- US-A- 4 773 170
- US-A- 4 969 277

## Beschreibung

Die Erfindung betrifft einen Verbandsschuh zum Tragen über Fußverbänden oder bei Fußverletzungen oder bei Fußwunden, welcher ein Sohlenteil mit an der Außenkante umlaufendem Klettverschlußmaterial und ein einteiliges, abwickelbares Obermaterialstück aufweist, das an der inneren Unterkante mit klettverschlußgeeigneten Verschlußbereichen zum Anschließen an das Klettverschlußmaterial des Sohlenteiles versehen ist, wobei das Obermaterialstück an seiner Teilung durch einen Klettverschluß verschließbar ist.

Der Bedarf an derartigen Verbandsschuhen ist erheblich und betrifft zum einen die Versorgung in der postoperativen Phase und zum anderen die ständige Anwendung bei Durchblutungsstörungen, offenen Stellen und dergleichen, insbesondere in der Geriatrie und bei schwerer Diabetes. Eine weitere Anwendung liegt im Kälteschutz und in der Stehhilfe für Rollstuhlfahrer und Rheumatiker. Gerade der hohe Anteil älterer Menschen an der Alterspyramide macht zunehmend gefäßchirurgische operative Maßnahmen sowie eine diabetesbedingte Ulkus-Versorgung notwendig, wobei Verbandsschuhe in den unterschiedlichsten Größen und Formen benötigt werden.

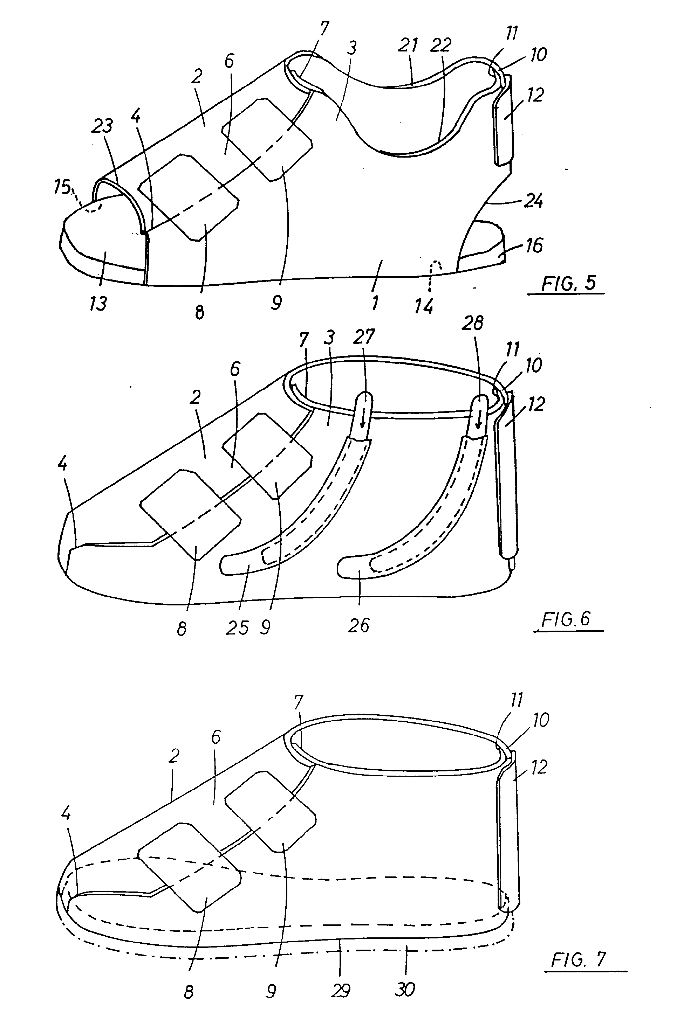

Ein gattungsgemäßer Verbandsschuh ist durch die US-A-4 300 294 bekannt. Dort ist das Obermaterialstück an seiner innenliegenden Unterkante mit einem aufgenähten Klettverschlußstreifen versehen, dessen Breite gleich der Sohlendicke ist. Ferner ist das Obermaterialstück längs der Kanten seiner Teilung mit angenähten schmalen Klettmaterialstreifen versehen, die das im Bereich der Teilung sich überlappende Obermaterialstück verschließen, wobei diese Teilung dort an der Schuhaußenseite (Fig. 6) oder am Spann (Fig. 7) angeordnet ist. Dieser bekannte Verbandsschuh kann zwar nachträglich an einen verbundenen Fuß angelegt und geschlossen werden, jedoch bieten die dortigen, verhältnismäßig schmalen Klettmaterialstreifen keine Möglichkeit, das Volumen und die Form des Verbandsschuhes den jeweiligen Gegebenheiten anzupassen.

Durch die US-A-4 773 170 und GB-A-2 168 234 sind ferner Verbandsschuhe bekannt, bei denen das Obermaterial aus zwei jeweils an den Längsrändern der Schuhsohle befestigten Teilen besteht, die hochgeklappt und mittels Klettverschlüssen im Fersenbereich und im Spannbereich miteinander verbunden werden. Diese bekannten Schuhe ermöglichen eine gewisse Volumenanpassung, jedoch ist eine Anpaßbarkeit insbesondere in der Höhe nur begrenzt möglich. Darüber hinaus sind diese Schuhe nur als rechter oder linker Schuh vorgegeben.

Durch die US-A-4 314 412 ist ferner ein Verbandsschuh bekannt, bei dem ein Obermaterialstück durch einen sohlenseitigen Schlitz hindurchgesteckt und über dem Spann und hinter der Ferse übereinander geschlagen und jeweils durch einen Klettverschluß geschlossen wird. Dieser Schuh ist zwangsläufig im Zehenbereich und im unteren Fersenbereich offen, so daß er nur unzureichenden Halt bietet.

Durch die US-A-2 236 367 und US-A-2 438 711 sind ferner Schuhe bekannt, an deren Sohle unterschiedliche Obermaterialteile angebracht werden können. Bei einer Verwendung dieser Schuhe als Verbandsschuhe müßte eine Vielzahl unterschiedlich großer und unterschiedlich geformter Obermaterialteile vorgehalten werden, was erhebliche Kosten verursachen würde.

Der Erfindung liegt die Aufgabe zugrunde, einen Verbandsschuh bereitzustellen, der hohen Tragekomfort und weitgehende Variabilität bei niedrigen Herstellungskosten sicherstellt.

Die Lösung hierfür besteht in einem Verbandsschuh, der dadurch gekennzeichnet ist, daß das Obermaterialstück im Fersenbereich oder an der Schuhinnen- oder Schuhaußenseite geteilt ist, daß das Obermaterialstück einen Einschnitt aufweist, der bis nahe an die zusammenhängende Unterkante des Obermaterialstückes heranreicht, daß das Obermaterialstück an seiner Außenseite klettverschlußfähig ist und durch aufgesetzte Klettmaterialstreifen im Bereich des Einschnittes und im Fersenbereich individuell verschließbar ist, daß die Verschlußbereiche am Obermaterialstück einen in Anpassung an das Sohlenteil abtrennbaren Zuschnittsbereich umfassen und daß das Obermaterialstück nach Anpassen des Schuhes zurechtschneidbar ist.

Ein derartiger Verbandsschuh ist im Fersenbereich, im Bereich des Einschnittes, der bevorzugt im Spannbereich liegt, und an der Befestigungsstelle zwischen Sohle und Obermaterialstück den jeweiligen medizinischen oder orthopädischen Erfordernissen anpaßbar, wobei in Folge der Trennbarkeit von Sohlenteil und Obermaterialstück mit einem einzigen Sohlenteil und vorzugsweise auch mit einem einzigen Obermaterialstück durch einfaches Wenden des Sohlenteils die Einsatzfähigkeit als rechter oder als linker Schuh ermöglicht ist. Dies ist für die Werkzeugbevorratung und die Lagerhaltung von erheblicher Bedeutung, da in der Regel die genannten Verbandsschuhe nur als Einzelstück nachgefragt werden. Grundsätzlich kann die Teilung des einstückigen Obermaterialstückes von jeder am Sohlenteil ansetzenden Stelle der Außenkante ausgehen. Bevorzugt ist jedoch die Teilung im Fersenbereich, bei der durch verschieden große Überdeckungen beim Anlegen des Obermaterialstückes am Sohlenteil eine weitgehende Variation im Volumen des geschlossenen zusammengesetzten Verbandsschuhes erreicht werden kann.

Es ist jedoch ebenso möglich, einen Überdeckungsbereich seitlich innen oder seitlich außen vorzusehen, wobei bei gleicher Gesamtflächenform dann eine andere abgewickelte Form des Obermaterialstückes zustande kommt.

Eine noch weitergehende Variation des Volumens des zusammengesetzten Verbandsschuhes ist durch den von der Oberkante des Obermaterialstückes ausgehenden Einschnitt bis in die Nähe des Sohlenmaterials verwirklicht, der bei an das Sohlenteil angesetztem Obermaterialstück überlappt werden kann, so daß eine weitergehende Variation des Volumens des Verbandsschuhes je nach Fußdicke oder Verbandsdicke möglich ist. In bevorzugter Ausführung ist ein derartiger Einschnitt im Spannbereich vorgesehen.

Das Obermaterial ist auf der Außenseite klettverschlußfähig, das heißt, es besteht aus einem Schlingen aufweisenden Material, das mit Haken aufweisenden Klettverschlußteilen zusammenwirkt. Dies ermöglicht es, sowohl den Fersenbereich als auch die bevorzugt im Spannbereich liegenden, von der oberen Kante ausgehenden Einschnitte nach teilweisem Überlappen oder Überdecken mit Klettverschlüssen individuell zu verschließen. Eine weitere günstige Anpassungsmöglichkeit ist dadurch gegeben, daß im Befestigungsbereich, d. h. der an der Sohle anliegenden Kante des Obermaterialstückes, ein Zuschnittsbereich vorgesehen ist, der je nach Spannhöhe oder Fußdicke bei vorgegebener Sohlenlänge ein variables Anpassen des Obermaterialstückes an das Sohlenteil sowie ein anschließendes Abschneiden entlang der Sohlenkante von überstehendem Material des Zuschnittsbereiches zuläßt.

Wenige unterschiedliche Größen der Einzelteile, etwa vier, sind aufgrund der weitgehenden Variabilität geeignet, alle üblichen Fußgrößen in zufriedenstellender Weise abzudecken.

Besonders vorteilhaft ist es fertigungstechnisch, wenn das Obermaterialstück in Abwicklung vollkommen eben ist. Nach einer ersten günstigen Ausführungsform ist das Obermaterialstück in Abwicklung symmetrisch geformt, so daß es ebenfalls mühelos für einen rechten oder linken Verbandsschuh verwendet werden kann. Eine Alternative bei asymmetrischer Form des Obermaterialstückes in Abwicklung besteht darin, daß es auf beiden Seiten gleiche Materialbeschaffenheit hat, so daß auf diese Weise die Verwendbarkeit für einen rechten oder linken Verbandsschuh möglich wird.

Die bevorzugte Formgebung für das Obermaterialstück besteht darin, daß es abgewickelt aus zwei bogenförmigen Teilen mit im Spannbereich größerer Breite besteht und seine Außenkante einen beide Teile begrenzenden, geschlossenen Bogen bildet.

Zur weiteren Variation und zur orthopädischen Anpassung können beim Zusammensetzen des Verbandsschuhes noch Einlagesohlen und Fersenkeile eingesetzt werden, die ausschließlich durch die Formgebung des Obermaterialstückes gehalten werden. Prinzipiell könnten jedoch auch diese mit außenliegendem Klettverschluß versehen werden, um eine Anbindung an das Obermaterialstück zu erhalten.

Weiterhin ist möglich, bei Feststehen des Einsatzes als rechter oder als linker Verbandsschuh das Sohlenteil zusätzlich mit einer rutschfesten Absatz- oder Sohlenauflage außen zu versehen, die einfach aufgeklebt werden kann.

Je nach Indikation kann es günstig sein, für besonders druckempfindliche Stellen am Fuß Ausschnitte oder Ausnehmungen im Obermaterialstück vorzusehen. Dies kann insbesondere die Knöchelbereiche, den Zehenbereich oder den Fersenbereich betreffen. In Sonderfällen können jedoch auch lochförmige Ausschnitte an beliebigen anderen Stellen vorgesehen werden. Es kann sich hierbei um von vornherein vorgesehene Ausnehmungen handeln oder solche, die nach Anpassung des Schuhes an den verletzten und verbundenen Fuß durch Ausschneiden oder Abschneiden an den Kanten hergestellt werden.

Eine weitere günstige Ausführung kann in einer gezielten Aussteifung des Obermaterialstückes liegen, die aus unmittelbar auf das Obermaterialstück aufgenähten oder aufgeklebten Bereichen aus einem verstärkten Material bestehen können, die jedoch insbesondere aus in aufgenähte Taschen eingesteckten Verstärkungsrippen hergestellt sein können. Für solche Verstärkungsrippen sind z. B. flache Spiralfedern geeignet. Dies ermöglicht eine Anpassung und Anwendung bei Fußdeformierungen.

Ein bevorzugtes Ausführungsbeispiel ist in der Zeichnung dargestellt. Hierin zeigen:
Fig. 1 ein Obermaterialstück in Abwicklung von außen,
Fig. 2 ein Obermaterialstück in Abwicklung von innen,
Fig. 3 einen fertigen Verbandsschuh in perspektivischer Ansicht,
Fig. 4 ein Sohlenteil, einen Fersenkeil und eine Einlagesohle in perspektivischer Ansicht,
Fig. 5 einen fertigen Verbandsschuh gemäß Fig. 3 mit ausgeschnittenen Bereichen,
Fig. 6 einen fertigen Verbandsschuh nach Fig. 3 mit Verstärkungseinlagen und
Fig. 7 einen fertigen Verbandsschuh mit Zuschnittsbereich am Obermaterial.

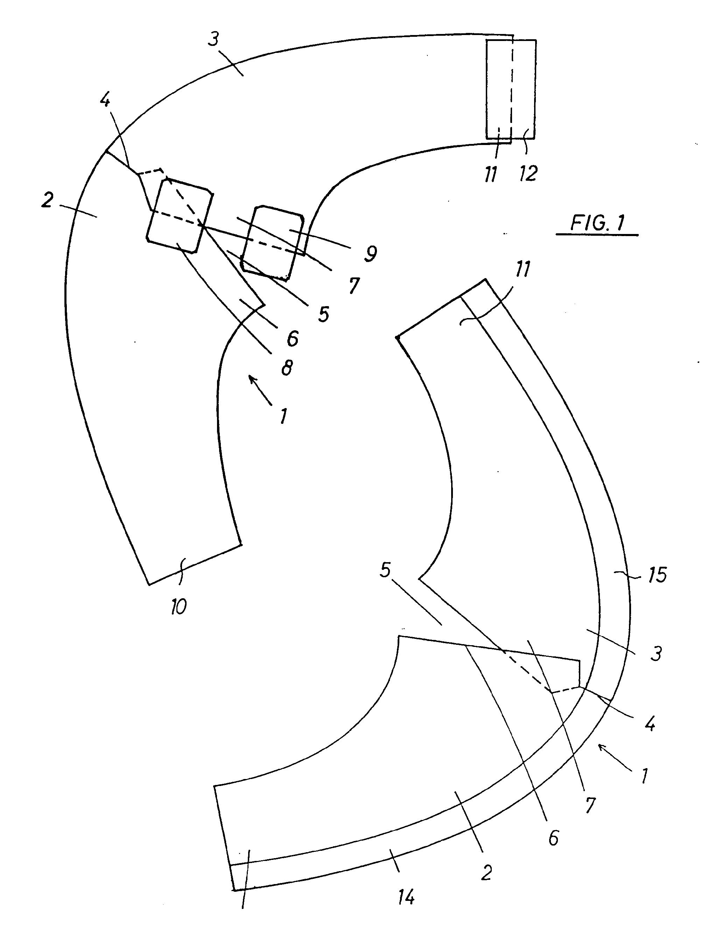

In Fig. 1 ist ein einteiliges Obermaterialstück 1 in Abwicklung dargestellt, das aus zwei ebenen, sichelähnlichen Teilen 2, 3 an einer Nahtstelle 4 zusammengesetzt ist, wobei sich zwei Kantenbereiche 6, 7 im Bereich eines Einschnittes 5 bereits in Abwicklung teilweise überlappen. Im Bereich dieses Einschnittes sind zwei auf dem oben liegenden Kantenbereich 7 befestigte Klettverschlüsse 8, 9 angebracht. Ein weiterer Klettverschluß 12 ist an einer der beiden hinteren Kanten 10, 11 außen aufgesetzt. Der Einschnitt 5 stellt den Spannbereich dar, die hinteren Kanten 10, 11 bilden später in Überlappung den Fersenbereich.

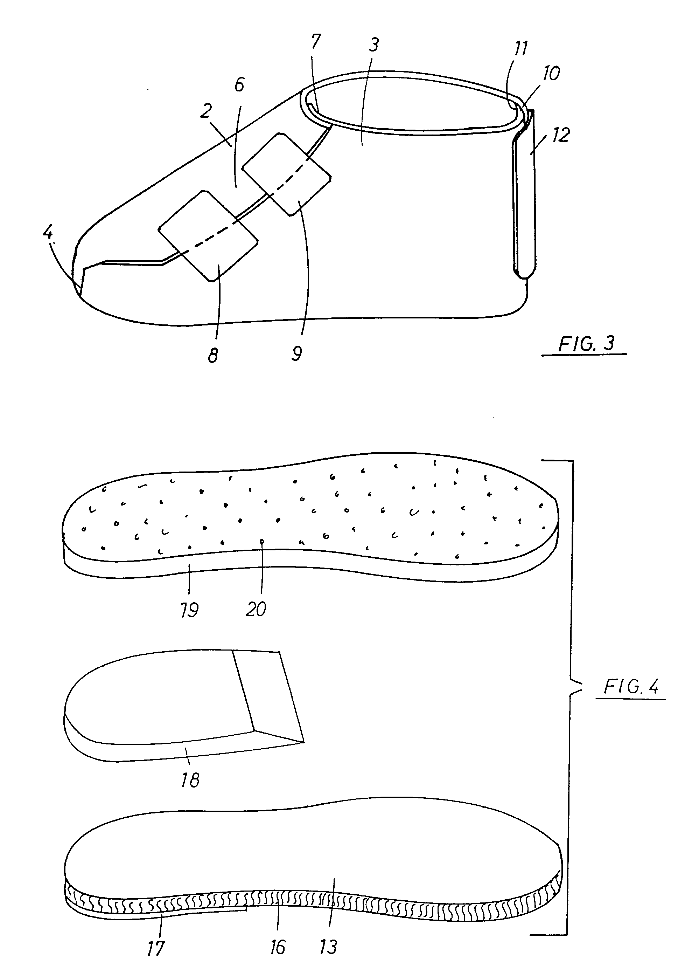

In Fig. 2 sind im wesentlichen die gleichen Einzelheiten wie in Fig. 1 erkennbar. Auch hier ist das Obermaterialteil 1 aus den zwei Stücken 2 und 3 an der Naht 4 so zusammengesetzt, daß sich ein Einschnitt 5 bildet, wobei sich die Kantenbereiche 6, 7 teilweise überdecken. Die Klettverschlüsse im Spannbereich sind hier nicht dargestellt. An der inneren Unterkante der Teile 2 und 3 sind jedoch klettverschlußfähige Bereiche 14, 15 aufgesetzt.

In Fig. 3 ist der zusammengesetzte Verbandsschuh zu erkennen, der aus den Obermaterialteilen 2, 3 besteht, die an der Nahtstelle 4 miteinander verbunden sind und bei dem der Kantenbreich 6 den Kantenbereich 7 im Spannbereich überlappt. Die Bereiche sind mit den Klettverschlüssen 8, 9 im Bereich der vorderen Kanten miteinander verbunden, der weitere Klettverschluß 12 verbindet die hinteren Kantenbereiche 10, 11 im Fersenbereich. Die Übermaterialstücke sind hier am nicht erkennbaren Sohlenteil rundum angesetzt.

In Fig. 4 ist ein Sohlenteil 13 dargestellt, das zum Beispiel aus geschlossen-porigem Schaumstoff bestehen kann, an dem außen ein umlaufender Klettverschluß 16 befestigt ist, der mit den Verschlußbereichen 14, 15 gemäß Fig. 2 zur Verbindung gebracht werden kann. Ein rutschfestes Absatzteil 17 ist für die Verwendung als linkes Sohlenteil, wie dargestellt, untergeklebt. Über dem Sohlenteil 13 ist ein in den Verbandsschuh einlegbarer Fersenkeil 18 und eine aus Weichschaumstoff bestehende Einlagesohle 19 mit Atmungsöffnungen 20 in richtiger Zuordnung zum Sohlenteil 13 dargestellt. Dieses kann in variabler Stärke verwendet zur besseren Anpassung des Verbandsschuhs dienen.

In Fig. 5 sind die entsprechenden Einzelheiten übereinstimmend mit Fig. 3 beziffert. Weiterhin sind als Einzelheiten Ausschnitte 21, 22 für die Fußknöchel, ein Ausschnitt 23 für die Zehen und ein Ausschnitt 24 für die Ferse erkennbar. Die Naht 4, die die beiden Hälften des Obermaterials zusammenhält, ist oberhalb des Zehenauschnitts 23 erkennbar.

In Fig. 6 sind alle Einzelheiten in Übereinstimmung mit Fig. 3 beziffert. Darüber hinaus sind zwei Taschen 25, 26 auf der Fußaußenseite erkennbar, in die Versteifungsrippen 27, 28 in Richtung der Pfeile eingesteckt werden können.

In Fig. 7 sind die gleichen Einzelheiten wie in Fig. 3 mit den gleichen Ziffern belegt. Das Sohlenteil 13 ist gestrichelt dargestellt, wobei die Unterkante des Obermaterials bündig mit der Unterkante 29 der Sohle abschließt, nachdem ein strichpunktiert dargestellter Zuschnittsbereich 30 vom Obermaterial abgetrennt worden ist. Für verschiedene Fußdicken können mehrere solcher Bereiche bereits durch nebeneinanderliegende Markierungen entlang der Unterkante vorgezeichnet sein, so daß ein Abtrennen überschüssigen Materials sowohl vor dem Zusammensetzen des Schuhs als auch bei angepaßtem Schuh erfolgen kann.

### Bezugszeichenliste

1 Obermaterialstück
2 Innenhälfte
3 Außenhälfte
4 Naht
5 Einschnitt
6 Kantenbereich (vorne)
7 Kantenbereich (vorne)
8 Klettverschluß
9 Klettverschluß
10 Kantenbereich (hinten)
11 Kantenbereich (hinten)
12 Klettverschluß
13 Sohlenteil
14 Verschlußbereich
15 Verschlußbereich
16 Klettverschluß
17 Absatzteil
18 Fersenkeil
19 Einlagesohle
20 Atmungsöffnung
21 Knöchelausschnitt
22 Knöchelausschnitt
23 Zehenausschnitt
24 Fersenausschnitt
25 Einstecktasche
26 Einstecktasche
27 Verstärkungsleiste
28 Verstärkungsleiste
29 Unterkante
30 Zuschnittsbereich

## Patentansprüche

1. Verbandsschuh zum Tragen über Fußverbänden oder bei Fußverletzungen oder bei Fußwunden, welcher ein Sohlenteil (13) mit an der Außenkante umlaufendem Klettverschlußmaterial (16) und ein einteiliges, abwickelbares Obermaterialstück (1) aufweist, das an der inneren Unterkante mit klettverschlußgeeigneten Verschlußereichen (14, 15) zum Anschließen an das Klettervschlußmaterial (16) des Sohlenteiles (13) versehen ist, wobei das Obermaterialstück (1) an seiner Teilung durch einen Klettverschluß verschließbar ist, dadurch gekennzeichnet, daß das Obermaterialstück (1) im Fersenbereich oder an der Schuhinnen- oder außenseite geteilt ist, daß das Obermaterialstück (1) einen Einschnitt (5) aufweist, der bis nahe an die zusammenhängende Unterkante des Obermaterialstückes heranreicht, daß das Obermaterialstück (1) an seiner Außsenseite klettverschlußfähig ist und durch aufgesetzte Klettmaterialstreifen (8, 9, 12) im Bereich des Einschnittes (5) und im Fersenbereich individuell verschließbar ist, daß die Verschlußbereiche (14, 15) am Obermaterialstück (1) einen in Anpassung an das Sohlenteil (13) abtrennbaren Zuschnittsbereich (30) umfassen und daß das Obermaterialstück (1) nach Anpassen des Schuhes zurechtschneidbar ist.

2. Verbandsschuh nach Anspruch 1, dadurch gekennzeichnet, daß der Einschnitt (5) im Spannbereich des Verbandsschuhes liegt.

3. Verbandsschuh nach Anspruch 1 oder 2, dadurch gekennzeichnet, daß das Obermaterialstück (1) in Abwicklung aus ebenem Material besteht.

4. Verbandsschuh nach Anspruch 3, dadurch gekennzeichnet, daß das Obermaterialstück (1) in Abwicklung symmetrisch geformt ist.

5. Verbandsschuh nach den Ansprüchen 1, 2 oder 3, dadurch gekennzeichnet, daß das Obermaterialstück (1) in Abwicklung asymmetrisch geformt ist und auf beiden Seiten gleiche Materialbeschaffenheit aufweist.

6. Verbandsschuh nach einem der Ansprüche 4 oder 5, dadurch gekennzeichnet, daß das Obermaterialstück (1) in Abwicklung aus zwei bogenförmigen Teilen (2, 3) mit im Spannbereich größerer Breite besteht und seine Außenkante einen geschlossenen Bogen bildet.

7. Verbandsschuh nach einem der Ansprüche 1 bis 6, dadurch gekennzeichnet, daß das Obermaterialstück (1) zumindest einen Ausschnitt (21, 22) im Bereich der Fußknöchel aufweist.

8. Verbandsschuh nach einem der Ansprüche 1 bis 7, dadurch gekennzeichnet, daß das Obermaterialstück (1) eine Ausnehmung (23) im Zehenbereich aufweist.

9. Verbandsschuh nach einem der Ansprüche 1 bis 8, dadurch gekennzeichnet, daß das Obermaterialstück (1) eine Ausnehmung (24) im Bereich der Ferse aufweist.

10. Verbandsschuh nach einem der Ansprüche 1 bis 9, dadurch gekennzeichnet, daß das Obermaterialstück (1) innen- oder außenliegende längliche Taschen (25, 26) zum Einstecken von Verstärkungsteilen (27, 28) aufweist.

## Claims

1. Bandage shoe to be worn over foot bandages or in cases of injury or wound to the foot, which has a sole section (13) with hook and eye fastener material (16) running round its outside edge, and a one-part roll-off upper material piece (1), equipped on its inner lower edge with closure areas suitable for closure by hook and eye fastener (14,15) for attaching to the hook and eye fastener material (16) of the sole section (13), in which the upper material piece (1) is closable along its division by hook and eye fastener, characterised in that the upper material piece is divided in the area of the heel or on the inner or outer side of the shoe, in that there is an incision (5) in the upper material piece (1) reaching almost to the connecting, lower edge of the upper material piece, in that the upper material piece (1) is closable, on its exterior edge, by hook and eye fastener and may be separately closed in the area of the incision (5) and in the area of the heel using applied strips of hook and eye fastener material (8) (9) (12), in that the closure areas (14,15) on the upper material piece (1) include a separable cut-and-fit area (30) matching the sole section (13) and in that the upper material piece (1) may be cut to the right dimensions after fitting of the shoe.

2. Bandage shoe according to Claim the incision (5) is located in the stretch area of the bandage shoe.

3. Bandage shoe according to Claim 1 or 2, characterised in that the upper material piece consists when unrolled of flat material.

4. Bandage shoe according to Claim 3, characterised in that the upper material piece (1) when unrolled is symmetrical in shape.

5. Bandage shoe according to Claims 1, 2 or 3, characterised in that the upper material piece (1), when unrolled, is asymmetrical in shape and has on both sides material of similar composition.

6. Bandage shoe according to one of Claims 4 or 5, characterised in that the upper material piece (1) when unrolled consists of two arc-shaped sections (2) and (3) with greater breadth in the stretch area, and that its outer edge forms a closed arc.

7. Bandage shoe according to one of Claims 1 to 6, characterised in that the upper material piece (1) has at least one cut-out (21) (22) in the area of the ankle bones.

8. Bandage shoe according to one of Claims 1 to 7, characterised in that the upper material piece (1) has a cut-out (23) in the toe area.

9. Bandage shoe according to one of Claims 1 to 8, characterised in that the upper material piece (1) has a cut-out (24) in the area of the heel.

10. Bandage shoe according to one of Claims 1 to 9, characterised in that the upper material piece (1) has interior or exterior longitudinal pockets (25,26) for insertion of reinforcing elements (27,28).

## Revendications

1. Chaussure pour pied bandé, à porter sur des bandages de pied ou en cas de blessures du pied ou de plaies du pied, qui présente une partie semelle (13) munie d'un matériau à fermeture adhésive ou par accrochage (16) sur le pourtour de l'arête extérieure et une pièce de matériau de dessus (1), d'un seul tenant et déroulable, pourvue sur l'arête inférieure intérieure de zones de fermeture (14,15) appropriées pour fermeture par accrochage, en vue d'opérer le raccordement au matériau à fermeture par accrochage (16) de la partie semelle (13), la pièce de matériau de dessus (1) étant susceptible d'être fermée sur une ligne de partage, au moyen d'une fermeture par accrochage, caractérisée en ce que la pièce de matériau de dessus (1) est divisée, dans la zone de talon ou en face intérieure ou extérieure de la chaussure, que la pièce de matériau de dessus (1) présente une entaille (5), arrivant jusqu'à proximité de l'arête inférieure, cohérente, de la pièce de matériau de dessus, que la pièce de matériau de dessus (1) est susceptible de constituer une fermeture par accrochage sur sa face extérieure et d'être fermée au moyen de bandes de matériau d'accrochage (8,9,12) que l'on applique, individuellement, dans la zone de l'entaille (5) et dans la zone de talon, que les zones de fermeture (14,15) sur la pièce de matériau de dessus (1) comprennent une zone de découpe (30), séparable lors de l'adaptation à la partie semelle (13) et qu'après adaptation de la chaussure, la pièce de matériau de dessus (1) peut être découpée à la mesure.

2. Chaussure pour pied bandé selon la revendication 1, caractérisée en ce que l'entaille (5) est située dans la zone de serrage de la chaussure.

3. Chaussure pour pied bandé selon la revendication 1 ou 2, caractérisée en ce que la pièce de matériau de dessus (1) est formée par développement d'un matériau plan.

4. Chaussure pour pied bandé selon la revendication 3, caractérisée en ce que la pièce de matériau de dessus (1) est de forme développée symétrique.

5. Chaussure pour pied bandé selon les revendications 1, 2 ou 3, caractérisée en ce que la pièce de matériau de dessus (1) est de forme développée asymétrique, présentant des caractéristiques de matériau identiques sur les deux faces.

6. Chaussure pour pied bandé selon l'une des revendications 4 et 5, caractérisée en ce que la pièce de matériau de dessus (1) est de forme développée composée de deux parties cintrées (2,3) de plus grande largeur dans la zone de serrage et son arête extérieure forme un arc fermé.

7. Chaussure pour pied bandé selon l'une des revendications 1 à 6, caractérisée en ce que la pièce de matériau de dessus (1) présente au moins une découpe (21,22) dans la zone de la cheville.

8. Chaussure pour pied bandé selon l'une des revendications 1 à 7, caractérisée en ce que la pièce de matériau de dessus (1) présente un évidement (23) dans la zone des orteils.

9. Chaussure pour pied bandé selon les revendications 1 à 8, caractérisée en ce que la pièce de matériau de dessus (1) présente un évidement (24) dans la zone du talon.

10. Chaussure pour pied bandé selon les revendications 1 à 9, caractérisée en ce que la pièce de matériau de dessus (1) présente des poches allongées intérieures ou extérieures (25,26) pour enficher des pièces de renforcement (27,28).
